# EUROPÄISCHE PATENTSCHRIFT

(11) **EP 1 784 476 B1**
(45) Veröffentlichungstag und Bekanntmachung des Hinweises auf die Patenterteilung: **16.01.2008**
(21) Anmeldenummer: 05770210.2
(22) Anmeldetag: 06.08.2005
(51) Int. Cl.: C10G 45/40, C07C 6/04, C07C 45/50

(54) **VERFAHREN ZUR HERSTELLUNG VON C5-ALDEHYDEN UND PROPEN AUS EINEM 1-BUTEN-UND 2-BUTEN-HALTIGEN C4-STROM**
METHOD FOR THE PRODUCTION OF C5 ALDEHYDES AND PROPENE FROM A C4 STREAM CONTAINING 1-BUTENE AND 2-BUTENE
PROCEDE POUR PRODUIRE DES ALDEHYDES C5 ET DU PROPENE A PARTIR D'UN FLUX C4 CONTENANT DU 1-BUTENE ET DU 2-BUTENE

(30) Priorität: 27.08.2004 DE 102004041850
(43) Veröffentlichungstag der Anmeldung: 16.05.2007
(73) Patentinhaber: BASF Aktiengesellschaft, 67056 Ludwigshafen (DE)
(72) Erfinder: SIGL, Marcus, 68167 Mannheim (DE); SCHUBERT, Markus, 67063 Ludwigshafen (DE); STEPHAN, Jürgen, 68163 Mannheim (DE); PAPP, Rainer, 67346 Speyer (DE); POPLOW, Frank, 67065 Ludwigshafen (DE)
(86) Internationale Anmeldenummer: PCT/EP2005/008561
(87) Internationale Veröffentlichungsnummer: WO 2006/024366

(56) Entgegenhaltungen:
- WO-A-20/05009934
- US-A- 5 877 365
- US-A- 5 898 091
- US-A1- 2003 153 791

## Beschreibung

Die vorliegende Erfindung betrifft ein Verfahren zur Herstellung von C₅-Aldehyden und Propen aus einem 1-Buten- und 2-Buten-haltigen C₄-Strom.

Zur wirtschaftlichen Nutzung von C₄-Strömen, wie sie beispielsweise aus Crackverfahren oder durch Dehydrierung von Butanen erzeugt werden können, sind bereits verschiedene Verfahren bekannt. Dies Ausgangsströme enthalten typischerweise größere Mengen an 1,3-Butadien, 1-Buten und 2-Butenen. Daneben sind oftmals nennenswerte Anteile an Butanen, sowie im Falle von Crackverfahren Isobuten enthalten. Um einen möglichst wirtschaftlichen Prozess zu erzielen, müssen die einzelnen Komponenten jeweils zu möglichst hochpreisigen Verkaufsprodukten umgewandelt werden, ohne dass die anderen Komponenten dadurch beeinträchtigt werden. Besonders vorteilhaft ist hierbei noch die ganze oder teilweise Umwandlung von einer C₄-Komponente in eine andere C₄-Komponente, die einer wirtschaftlich attraktiveren Verwendung zugeführt werden. Hierzu sind im allgemeinen komplexe, mehrstufige Verfahren erforderlich, in denen die Verarbeitung der einzelnen C₄-Komponenten schrittweise erfolgt. Solche Verfahren sind z.B. in der DE-A-10118634, EP-A-742 195 und 742 234 beschrieben.

Reines 1,3-Butadien stellt einen gefragten Monomerbaustein dar. Reines 1-Buten ist ebenfalls ein hochpreisiger Monomerbaustein, findet aber auch nach Hydroformylierung zu Valeraldehyd und anschließender Aldolkondensation und Hydrierung zu Propylheptanol einen wirtschaftlich bedeutenden Auslass als Weichmacherkomponente und Tensidatkohol. Isobuten dient nach Polymerisation zu Polyisobuten als Ausgangsmaterial für Kraft- und Schmierstoffadditive, nach Veretherung mit Methanol zu MTBE als Treibstoffadditiv sowie nach Dimerisierung zu Diisobuten und anschließender Hydrierung als klopffestes Alkylatbenzin. Dagegen ist die direkte chemische Umsetzung von 2-Butenen technisch bislang unbedeutend. Sinnvoll ist hier eine Olefinmetathese mit Ethen, die 2-Butene in den wertvollen Olefinbaustein Propen umwandelt.

Aufgabe der vorliegenden Erfindung war es, ein Verfahren zu entwickeln, das eine möglichst vollständige und möglichst wirtschaftliche Nutzung eines C₄-Stroms zur Herstellung von Propen und C₅-Aldehyden ermöglicht.

Demgemäß wurde ein Verfahren zur Herstellung von C₅-Aldehyden und Propen aus einem 1-Buten- und 2-Butene-haltigen C₄-Strom, der bis zu 1000 Gew.-ppm 1,3-Butadien enthält (C₄-Eingangsstrom) gefunden, umfassend
a) eine Hydroformylierungsstufe, bei der man den C₄--Eingangsstrom in Gegenwart eines üblichen Hydroformylierungskatalysators mit Wasserstoff und Kohlenmonoxid in Kontakt bringt und die dabei gebildeten C₅-Aldehyde und den dabei gebildeten 2-Buten-reichen C₄-Strom anschließend voneinander trennt und
b) eine Metathesestufe, bei der man den in der Hydroformylierungsstufe gebildeten 2-Buten-reichen C₄-Strom in Gegenwart eines üblichen Metathesekatalysators mit Ethen in Kontakt bringt und von dem dabei gebildeten Propen-haltigen Kohlenwasserstoffstrom das Propen abtrennt.

Als C₄-haltige Ströme eignen sich insbesondere sog. Raffinate (Raffinat I oder II). Solche Raffinate I sind nach 3 verschiedenen Methoden herstellbar:
Bei der ersten Methode stellt man den C₄-Eingangsstrom bereit, indem man
Ia) in Schritt la Naphtha oder sonstige Kohlenwasserstoffverbindungen einem Steamcracking- oder FCC-Prozess unterwirft und aus dem dabei gebildeten Stoffstrom eine C₄-Olefin-Mischung abzieht, die 1-Buten, 2-Buten, und mehr als 1000 Gew.-ppm Butadiene und ggf. Butine und ggf.Isobuten enthält
IIa) aus der in Schritt la gebildeten C₄- eine Olefin-Mischung einen im wesentlichen aus, 1-Buten, 2-Butenen und ggf. Butanen und ggf. Isobuten bestehenden C₄-Kohlenwasserstoffstrom (Raffinat I) herstellt, indem man mittels Selektivhydrierung die Butadiene und Butine zu Butenen oder Butanen hydriert oder die Butadiene und Butine durch Extraktivdestillation soweit entfernt, dass der Gehalt an 1,3-Butadien maximal 1000 Gew.-ppm beträgt.

Bei der zweiten Methode stellt man den C₄-Eingangsstrom bereit, indem man
lb) in Schritt lb aus einem Butane enthaltenden Kohlenwasserstoffstrom durch Dehydrierung und nachfolgende Reinigung eine C₄-Olefin-Mischung herstellt, die Isobuten, 1-Buten, 2-Buten, und mehr als 1000Gew.-ppm Butadiene und ggf. Butine und ggf. Butanen enthält
IIb) aus der in Schritt IB gebildeten C₄-Olefin-Mischung einen im wesentlichen aus Isobuten, 1-Buten, 2-Butenen und ggf. Butanen bestehenden C₄-Kohlenwasserstoffstrom (Raffinat I) herstellt, indem man mittels Selektivhydrierung die Butadiene und Butine zu Butenen oder Butanen hydriert oder die Butadiene und Butine durch Extraktivdestillation soweit entfernt, dass der Gehalt an 1,3-Butadien maximal 1000Gew.-ppm beträgt.

Bei der dritten Methode stellt man den C₄-Eingangsstrom (in Form von Raffinat II) bereit, indem man
Ic) aus Methanol durch Dehydrierung eine C₄-Olefin-Mischung herstellt (MTO-Verfahren) die 1-Buten, 2-Buten, und ggf. Butadiene, Alkine und ggf. Butane enthält und
Ic) die C₄-Olefin-Mischung durch Destillation, Selektivhydrierung oder Extraktivdestillation von Butadienen oder Alkinen befreit.

Raffinat II weist im wesentlichen die gleiche Zusammensetzung wie Raffinat I auf, bis auf die Tatsache, dass Raffinat II geringere Mengen an Isobuten enthält. Typischerweise weist Raffinat II Mengen von weniger als 10Gew.-%, bevorzugt weniger als 5Gew.-% Isobuten auf.

Aus dem Raffinat I kann das Raffinat II hergestellt werden, indem man aus dem Raffinat I den wesentlichen Anteil des Iso-Butens durch bekannte chemische, physikalischchemische oder physikalische Methoden abtrennt.

Hierzu gibt es prinzipiell drei grundlegend verschiedene Möglichkeiten: a) eine destillative Abtrennung, b) eine Abtrennung durch Veretherung/Extraktion und c) die Direktpolymerisation zu Polyisobuten.

Die Destillation (Methode a) findet in einem dafür geeigneten Apparat statt, z.B. einer Glockenbodenkolonne, Füllkörperkolonne, Packungskolonne oder Trennwandkolonne. Bevorzugt ist die Destillationskolonne mit 20 bis 80 theoretischen Trennstufen ausgeführt. Das Rücklaufverhältnis beträgt im Allgemeinen 5 bis 50. Die Destillation wird im Allgemeinen bei einem Druck von 5 bis 20 bar durchgeführt.

Bedingt durch den niedrigen Siedepunkt des Isobutens und 1-Butens im Vergleich zu 2-Butenen und n-Butan wird der Kopfstrom hauptsächlich Isobuten und 1-Buten, der Sumpfstrom hauptsächlich 2-Butene und n-Butan enthalten. Der Gehalt an Leichtsiedern (Isobuten und 1-Buten) im Sumpfstrom ist kleiner 40%, bevorzugt kleiner 30% und besonders bevorzugt 5 - 20%. Der Gehalt an Hochsiedern (2-Butene und n-Butan) im Kopfstrom ist kleiner 40%, bevorzugt kleiner 30% und besonders bevorzugt 5 bis 20%.

Bei Methode b) geht man üblicherweise so vor, dass man Raffinat I mit einem Alkylalkohol, bevorzugt einem C₁- bis C₄-Alkylalkohol und einem üblichen Katalysator für die Bildung von Alkyl-tert-butylether in Kontakt bringt und den gebildeten Alkyl-tert-butylether von dem verbleibenden Raffinat II abtrennt. Besonders bevorzugte Alkohole: MeOH, BuOH

Die Veretherung erfolgt bevorzugt in Gegenwart eines sauren lonentauschers in einer dreistufigen Reaktorkaskade, in der geflutete Festbettkatalysatoren von oben nach unten durchströmt werden, wobei die Reaktor-Eingangstemperatur 0 bis 60°C, vorzugsweise 10 bis 50°C, die Ausgangstemperatur 25 bis 85°C, vorzugsweise 35 bis 75°C, der Druck 2 bis 50 bar, vorzugsweise 3 bis 20 bar und das Verhältnis von Alkohol zu Isobuten 0,8 bis 2,0, vorzugsweise 1,0 bis 1,5 beträgt.

Bei Methode c) geht man üblicherweise so vor, dass man Raffinat I mit einem üblichen Katalysator für die Polymerisation von Isobuten in Kontakt bringt und das gebildete Polyisobutylen von dem verbleibenden C₄-Eingangsstrom abtrennt. Als Katalysator bevorzugt eingesetzt wird ein homogener oder heterogener Katalysator aus der Klasse der Bronstedt- oder Lewis-Säuren. Bevorzugt handelt es sich bei dem Katalysator um Bortrifluorid

Falls es die Wirtschaftlichkeit des Gesamtprozesses verbessert, kann optional entferntes Isobuten auch einer Skelettisomerisierung zugeführt werden - auch eine Kombination von Destillation und Skelettisomerisung in einer Art Reaktivdestillation ist an dieser Stelle möglich - um die Mengen an linearen Olefinen zu erhöhen und damit die Ausbeuten in der 1-Buten verwertenden Stufe bzw. der Metathese zu steigern.

Vorzugsweise wird die Extraktivdestillation in Schritt IIa, IIb oder IIc mit einem Butadien-selektiven Lösungsmittel durchgeführt, ausgewählt aus der Klasse polaraprotischer Lösemittel, wie Aceton, Furfural, Acetonitril, Diemethylacetamid, Dimethylformamid und N-Metylpyrrolidon.

Die Selektivhydrierung in Schritt IIa, IIb oder IIc kann zu einer weitgehenden Reduktion von Diolefinen bzw. acetylenischen Verbindungen eingesetzt werden, da diese Verbindungen die nachfolgende Verfahrensstufen beeinträchtigen würden. Zudem kann durch die selektive Hydrierung einer größeren Menge von 1,3-Butadien auch die Menge an linearen Monoolefinen beträchtlich erhöht werden, was die Produktioriskapazität nachfolgender Stufen erhöht. Durch geeignete Katalysatoren sowie Fahrweisen (z.B. H₂-Angebot) lässt sich das 1-Buten zu 2-Buten-Verhältnis in der Selektivhydrierung innerhalb gewisser Grenzen steuern (sog. Hydroisomerisierung). Da insbesondere für das 1-Buten attraktive wirtschaftliche Verwertungsmöglichkeiten gegeben sind, werden 1-Buten zu 2-Buten-Verhältnisse von wenigstens 1:3, bevorzugt von wenigstens 2:3, besonders bevorzugt von mehr als 1:1 angestrebt. Vorzugsweise wird der Teilschritt Selektivhydrierung in flüssiger Phase an einem Metall, ausgewählt aus der Gruppe Nickel, Palladium und Platin, auf einem Träger, vorzugsweise Palladium auf Aluminiumoxid, bei einer Temperatur von 20 bis 200°C, einem Druck von 1 bis 50 bar, einer Volumengeschwindigkeit von 0,5 bis 30 m³ Frischfeed pro m³ Katalysator pro Stunde und einem Verhältnis von Recycle zu Zustrom von 0 bis 30 mit einem Molverhältnis von Wasserstoff zu Diolefinen von 0,5 bis 50 durchgeführt.

Sofern der Gehalt an 1,3-Butadien in der gemäß Schritt Ia oder Schritt Ib gewonnenen C₄-Olefin-Mischung 5 Gew.-% oder mehr beträgt, senkt man üblicherweise den Gehalt an 1,3-Butadien mittels Extraktivdestillation auf einen Gehalt zwischen1000 Gew.-ppm und 5 Gew.-% ab und senkt den Gehalt an 1,3-Butadien anschließend mittels Selektivhydrierung weiter auf 1000Gew.-ppm oder weniger ab.

Bevorzugt weist der C₄-Eingangsstrom ein Verhältnis 1-Buten zu 2-Butene von 3 : 1 bis 1 : 3 auf.

Der Gehalt an 1,3-Butadien beträgt bevorzugt weniger als 300, besonders bevorzugt weniger als 100 Gew.-ppm.

Im Allgemeinen enthält der C₄-Eingangsstrom 2 bis 50 Gew.-% Butane, 15 bis 80 Gew.% 2-Butene und 20 und 60 Gew.-% 1-Buten, 20 bis 1000 Gew.-ppm Butadiene und 0 bis 50 Gew.-% Isobuten.

Die Hydroformylierungstufe kann im Allgemeinen in der für den Fachmann üblichen und bekannten Art und Weise durchgeführt werden. Eine gute Übersicht mit zahlreichen weiteren Literaturstellen findet sich beispielsweise in M. Beller et al., Journal of Molecular Catalysis A, 104, 1995, Seiten 17 bis 85 oder in Ullmann's Encyclopedia of Industrial Chemistry, 6th edition, 2000 electronic release, Chapter "ALDEHYDES, ALIPHATIC AND ARALIPHATIC - Saturated Aldehydes". Die dort gegebenen Informationen ermöglichen es dem Fachmann, sowohl die linearen als auch die verzweigten Alkene zu hydroformylieren.

In der Hydroformylierungsstufe wird übergangsmetallkatalysiert aus 1-Buten unter Zusatz von Synthesegas (CO:H₂ von 3:1 bis 1:3, bevorzugt 1,5 : 1 bis 1:1,15) Valeraldehyd (n-Pentanal) hergestellt.

Als Katalysatoren für die Hydroformylierungsreaktion werden in der Regel RhodiumKomplexe mit Phosphor-haltigen Liganden eingesetzt. Typischerweise handelt es sich bei den Phosphor-haltigen Liganden um ein mono- oder di-Phosphan, bevorzugt ein Triarylphosphan, besonders bevorzugt Triphenylphosphan. Durchgeführt wird die Hydroformylierungsreaktion üblicherweise bei Temperaturen von 50 bis 150°C, bevorzugt von 70 bis 120 °C und Drücken von 5 bis 50 bar, bevorzugt 10 bis 30 bar.

Der C4-Strom nach der Hydroformylierungsstufe (auch ''2-Buten-reicher C₄-Strom'' genannt) enthält üblicherweise 3 bis 70 Gew.-% Butane, 22 bis 90 Gew.-% 2-Butene, 20 bis 1000 Gew.-ppm 1,3-Butadien, 0 bis 10 Gew.% 1-Buten und 0 bis 65 Gew.-% Isobuten.

Das Verhältnis 1-Buten zu 2-Buten im 2-Buten-reichen C₄-Strom beträgt üblicherweise 1 : 3 bis 1 : 60.

Der 2-Buten-reiche C₄-Strom enthält bevorzugt weniger als 300 ppm, besonders bevorzugt weniger als 100 ppm 1,3-Butadien.

Der Umsatz der 1-Butene in dieser Verfahrensstufe ist bevorzugt größer als 80%, der absolute 1-Butene-Gehalt im 2-Buten-reichen C₄-Strom ist bevorzugt kleiner als 5%. Das Verhältnis von 1-Buten zu 2-Buten ist im 2-Buten-reichen C₄-Strom kleiner als 1:3, bevorzugt kleiner als 1:5.

Falls die Abtrennung des Isobutens nicht schon aus dem Cₐ-Eingangsstrom erfolgt ist, kann die Isobutenabtrennung der Hydroformylierungsstufe nachgeschaltet werden. Dies ist für die Varianten a und c bevorzugt. Hierfür eignen sich die gleichen Methoden, wie sie vorstehend bei der Herstellung von Raffinat I aus Raffinat II beschrieben sind.

Für eine hohe Ausbeute an Propen in der Metathesestufe, ist im Regelfall zunächst noch eine zusätzliche Reinigung erforderlich, die Spuren von Oxygenaten ggf. auch Acetylenen und Dienen abreichert. Die Gehalte an Oxygenaten, beispielsweise Wasser, Aceton oder Ethanol, sollten nach der Reinigungsstufe in Summe weniger als 100 ppm, bevorzugt weniger als 50 ppm, besonders bevorzugt weniger als 10 ppm betragen. Die Gehalte an Diolefinen sollten weniger als 300, bevorzugt weniger als 150 ppm, besonders bevorzugt weniger als 100 ppm betragen. Die Reinigung des 2-Buten-reichen C₄-Strom besteht aus einer oder mehreren Stufen und kann auch Druckwechselverfahren (Pressure-Swing-Adsorption) enthalten, bevorzugt ist jedoch zumindest ein adsorptives Verfahren. Bevorzugt erfolgt die Reinigung unmittelbar vor der Metathese, kann aber theoretisch auch ganz oder teilweise vor anderen Verfahrensstufen liegen. Optional kann die Reinigungsstufe auch eine Selektivhydrierung umfassen, die noch Restspuren von Diolefinen und Acetylen entfernt, die in der erste Selektivhydrierungsstufe nicht vollständig hydriert wurden oder sich in späteren Verfahrensstufen auch neu gebildet bzw. angereichert haben können. Bevorzugt enthält die Reinigungsstufe zumindest ein Adsorberbett auf Basis eines Aluminiumoxids oder eines Molsiebs zur Entfernung von Oxygenaten. Besonders bevorzugt ist eine Ausführungsform, bei der wenigstens zwei Adsorberbetten auf Basis eines Aluminiumoxids oder Molsiebs enthalten sind, die sich jeweils abwechselnd im Adsorptions- bzw. Regeneriermodus befinden. Bevorzugte Adsorbentien sind ein 13X-Molsieb bzw. hochoberflächige gamma-Aluminiumoxide (z. B. Selexsorb der Fa. Almatis).

Der 2-Buten-reiche C₄-Strom wird schließlich einer Metathesestufe zugeführt, um das 2-Buten in das höherwertige Monomer Propylen umzuwandeln. Hierzu erfolgt die stöchiometrische Zugabe (bezogen auf 2-Buten) von Ethylen oder Ethylen wird im Überschuss zugegeben. Eventuell im Strom enthaltenes 1-Buten oder Isobuten reagiert dabei zwar teilweise ebenfalls unter Bildung von höheren Olefinen (C₅ und C₆), diese können ausgeschleust oder aber in die Metathese rückgeführt werden, so dass kein oder nur ein geringer Nettoumsatz entsteht.

Falls der in der Metathesestufe gebildete Propen-haltigen Kohlenwasserstoffstrom C₅- und C₆-Olefine enthält, werden diese vom Propen abgetrennt und üblicherweise zumindest in dem Umfang in die Metathesestufe zurückführt, dass das molare Verhältnis der Summe der nicht zurückgeführten C₅- und C₆-Olefine zu Propen mit maximal 0,2 : 1 beträgt.

Auch nicht umgesetztes 2-Buten und Ethylen kann ggf. in die Metathesestufe zurückgeführt werden, da die Metathesereaktion eine Gleichgewichtsreaktion ist.

Für die Metathese kommen prinzipiell zwei verschiedene Katalysatortypen in Frage: a) Rheniumhaltige Katalysatoren, die bei Temperaturen im Bereich von 30 bis 150°C, bevorzugt im Bereich von 35 bis 110°C betrieben werden und b) W-haltige, Re-freie Katalysatoren, die in der Gasphase bei Temperaturen von 200 bis 600°C, bevorzugt von 220 bis 450°C betrieben werden.

Die Re-haltigen Katalysatoren enthalten bevorzugt wenigstens 1 Gew.% Re in oxidischer Form auf einem Träger, der zu wenigstens 75 Gew.-% aus einem hochoberflächigem Aluminiumoxid, ganz besonders bevorzugt gamma-Aluminiumoxid, besteht. Insbesondere bevorzugt sind Katalysatoren die einen Re-Gehalt von 5 bis 12 Gew.-% aufweisen und auf reinem gamma-Al203 geträgert sind. Die Katalysatoren können zur Steigerung der Aktivität auch noch zusätzlich Dotierstoffe enthalten, beispielsweise Oxiden von Nb, Ta, Zr, Ti, Fe, Mn, Si, Mo, W, Phosphat oder Sulfat. Die Katalysatoren haben bevorzugt Oberflächen von wenigstens 100 m²/g und Porenvolumen von wenigstens 0,3 ml/g. Geeignete Re-haltige Katalysatoren sind beispielsweise in DE-A 10 2004 009 804.2, DE-A 10 2004 009 805.0 oder DE-A 10 2004 009 803.4 beschrieben.

Geeignete W-haltige und Re-freie Katalysatoren enthalten bevorzugt wenigstens 3 wt% W, zumindest teilweise in oxidischer Form, auf einem Träger ausgewählt aus der Gruppe Aluminiumoxide, Alumosilikate, Zeolithe oder, bevorzugt SiO₂. Die Katalysatoren weisen bevorzugt eine Oberfläche von wenigstens 50 m²/g und ein Porenvolumen von wenigstens 0,3 ml/g aufweisen. Die Aktivität bzw. lsomerisierungsaktivität kann durch geeignete Dotierungen verbessert werden, beispielsweise Alkali- und Erdalkaliverbindungen, TiO₂, zrO₂, HfO₂, oder Verbindungen bzw. Elemente aus der Gruppe Ag, Sb, Mn, W, Mo, Zn, Si. Falls in der Metathese eine weitere Erhöhung des 1-Buten-Gehalts gewünscht ist, kann dem W-haltigen Katalystor auch ein Isomerisierungskontakt, beispielsweise ein Erdalkalioxid, untergemischt werden. Das führt dazu, dass in der Metathese neben Propen auch eine zusätzliche Menge an 1-Buten generiert wird, die wiederum nach destillativer Abtrennung der Verfahrenstufe b zugeführt werden kann und hier die Kapazität erhöht.

Es ist dem Fachmann bekannt, dass alle Arten von Metathesekatalysatoren regelmäßig oxidativ regeneriert werden müssen. Hierzu wird entweder ein Aufbau mit Festbetten und wenigstens zwei Reaktoren gewählt, von denen sich immer wenigstens ein Reaktor im Regenerationsmodus befindet, oder es kann alternativ ein Wanderbettverfahren ausgeübt werden, bei dem desaktivierter Katalysator ausgeschleust und extern regeneriert wird.

Speziell bei Verwendung eines Rhenium-haltigen Katalysators kommt auch die Ausführungsform der Reaktivdestillation in Betracht, bei der der Metathesekatalysator unmittelbar in der Destillationskolonne platziert wird. Diese Ausführungsform ist vor allem bei Anwesenheit größerer Mengen 1-Buten im Eingangsstrom gut geeignet. In diesem Fall werden nicht umgesetztes Ethylen, Propen und 1-Buten über Kopf genommen, die schwereren Olefine verbleiben zusammen mit dem Katalysator im Sumpf. Gegebenenfalls muss für eine Ausschleusung von Inerten, etwa Butanen, gesorgt werden. Diese spezielle Art der Reaktionsführung erlaubt die Umwandlung von 2-Buten in Propen ohne das der 1-Buten-Gehalt verändert wird.

Die Aufarbeitung des in der Metathesestufe gebildeten Propen-haltigen Kohlenwasserstoffstroms erfolgt bevorzugt mittels Destillation. Die destillative Auftrennung der kann in mehreren nacheinander geschalteten Destillationsstufen erfolgen oder der in der Metathesestufe gebildeten Propen-haltigen Kohlenwasserstoffstrom kann an geeigneter Stelle in die Trennapparatur, die im Steamcracker gebildete Kohlenwasserstoffmischung in einzelne Fraktionen aufspaltet, eingespeist werden.

Falls aus dem Propen in einem anschließenden Schritt ein Polymeres hergestellt werden soll, wird die das Propen weiter durch übliche Methoden gereinigt, dass sie der sog. polymer grade Spezifikation entspricht. Danach gelten folgende Obergrenzen für Verunreinigungen:

| | | |
|---|---|---|
| Propylen | > 99,5 | Gew.-% |
| Propan | < 5000 | Gew.-ppm |
| Methan | < 200 | Gew.-ppm |
| Ethan | < 300 | Gew.-ppm |
| Ethylen | < 30 | Gew.-ppm |
| Acetylen | < 1 | Gew.-ppm |
| Wasser | < 10 | Gew.-ppm |

### Beispiele:

Die folgenden Beispiele, denen Modellrechnungen zu Grund liegen, sollen Anordnungen zur Verwertung von C4-Strömen illustrieren. Bei den im Folgenden gestrichelt gezeichneten Komponenten in den Blockschemata handelt es sich jeweils um prinzipiell optionale Stufen. Diese werden in dem konkreten, zugehörigen Textbeispiel teilweise auch nicht genutzt.

### Beispiel 1

### Beispiel 1 wird durch Schema 1 weiter verdeutlicht.

Aus 400.000 jato eines Roh-C4-Stroms aus einem Naphtha-Cracker werden durch eine Butadien-Extraktion ca. 75.000 jato 1,3-Butadien entnommen. Die verbleibenden 325.000 jato haben nach der Selektivhydrierung folgende Zusammensetzung: 30,8% Isobuten, 30,8% 1-Buten, 30,8% 2-Butene, 80 ppm 1,3-Butadien, Rest Butane. Dieser Feed wird einer Stufe zur selektiven Hydroformylierung von 1-Buten zugeführt. Der 1-Buten-Umsatz liegt bei 90%, insgesamt je 3% des 1-Butens werden dabei zu 2-Buten bzw. Butan umgesetzt. Es werden knapp 130.000 jato Valeraldehyd produziert. Der verbleibende C4-Strom (ca. 240.000 jato) besteht aus 41,6% Isobuten, 4,2% 1-Buten, 42,7% 2-Butene, 100 ppm 1,3-Butadien und Rest Butane. Dieser Feed wird zunächst über ein 13X-Molsieb zur Entfernung von Oxygenatspuren geschickt und anschließend zusammen 51350 jato Ethylen der Metathesestufe zugeführt. Die Metathese läuft in der Gasphase an einem Festbettkatalysator, 10 wt% WO3 auf einem SiO2-Träger. Die Temperatur wird zum Ausgleich des fortschreitenden Aktivitätsverlusts nachgeregelt und beträgt 220°C bei start of run und 400°C bei end of run. Wenn nach ca. 2 bis 3 Wochen die Endtemperatur erreicht ist, wird der Katalysator oxidativ bei Temperaturen von ca. 550°C regeneriert. In dieser Zeit übernimmt ein zweiter, paralleler Reaktor (A/B-Fahrweise) die Produktion. Bei einem durchschnittlichen Gleichgewichtsumsatz von ca. 55% werden knapp 85000 jato Propen produziert. Der in der Metathesestufe gebildete Stoffstrom wird destillativ aufgearbeitet, entweder in einer separaten Destillationseinheit oder er wird hierzu der Destillationseinheit, die sich an einen Steamcracker anschließt, zugeführt. Bei seperater destillativer Aufarbeitung trennt man den Stoffstrom in wenigstens 4 verschiedene Fraktionen auf (s. Schema): a) eine hauptsächlich Ethylen enthaltene Fraktion (Fraktion a), b) eine hauptsächlich Propen enthaltende Fraktion (Fraktion b), c) eine C₄-, C₅- und C₆-Olfine enthaltene Fraktion, wobei es sich bei den C₄-Olefinen hauptsächlich um 2-Buten handelt und d) eine hauptsächlich leichtsiedende C₄-Kohlenwasserstoffe enthaltende Fraktion (Fraktion d). Fraktion a und c kann anschließend wieder in die Metathesestufe zurückgeführt werden (alternativ: Cracker).Fraktion (C4-Anteil ohne Berücksichtigung der Ströme:) c + d (184000 jato), wird zur Wiederaufarbeitung zum Cracker rückgeführt .

### Beispiel 2

### Beispiel 2 wird durch Schema 2 weiter verdeutlicht.

Aus 400.000 jato eines Roh-C₄-Stroms aus einem Naphtha-Cracker werden durch eine Butadien-Extraktion ca. 100.000 jato 1,3-Butadien entnommen. Die verbleibenden 300.000 jato haben nach der Selektivhydrierung folgende Zusammensetzung: 36,7%. Isobuten, 26,7% 1-Buten, 28,4% 2-Butene, 90 ppm 1,3-Butadien, Rest Butane. Dieser Feed wird einer Stufe zur selektiven Hydroformylierung von 1-Buten zugeführt. Der 1-Buten-Umsatz liegt bei 85%, insgesamt je 4% des 1-Butens werden dabei zu 2-Buten bzw. Butan umgesetzt. Es werden ca. 96.000 jato Valeraldehyd produziert. Der verbleibende C₄-Strom (ca. 237.000 jato) besteht aus 46,3% Isobuten, 5,1% 1-Buten, 38,1% 2-Butene, 110 ppm 1,3-Butadien und Rest Butane. In einer Stufe zur selektiven Polyisobutenbildung wird das Isobuten auf 5% abgereichert. Als saurer Katalysator dient BF₃. Dabei werden ca. 98.000 jato Polyisobuten gewonnen, der Reststrom (ca. 134.000 jato) besteht aus 5% Isobuten, 8,9% 1-Buten, 67,4% 2-Buten, 190 ppm 1,3-Butadien, Rest Butane.

Dieser Feed wird zunächst noch über eine Selektivhydrierungsstufe zur Reduktion des 1,3-Butadiengehalts auf 80 ppm geleitet. Der 1- zu 2-Buten-Gehalt wird in dieser Stufe nicht mehr verändert. Anschließend entfernt ein 13X-Molsieb Oxygenatspuren. Der C₄-Feed wird anschließend zusammen mit ca. 45000 jato Ethylen der Metathesestufe zugeführt. Die Metathese läuft in der Flüssigphase an einem Festbettkatalysator, 10 wt% Re₂O₇ auf einem gamma-Aluminiumoxid-Träger. Die Temperatur wird zum Ausgleich des fortschreitenden Aktivitätsverlusts nachgeregelt und beträgt 35°C bei start of run und 110°C bei end of run. Wenn nach ca. 1 Wochedie Endtemperatur erreicht ist, wird der Katalysator oxidativ bei Temperaturen von ca. 550°C regeneriert. In dieser Zeit übernimmt ein zweiter, paralleler Reaktor (A/B-Fahrweise) die Produktion. Bei einem durchschnittlichen Gleichgewichtsumsatz von ca. 63% werden rund 64.000 jato Propen produziert. Die Aufarbeitung des in der Metathesestufe gebildeten Stoffstroms erfolgt wie bei Schema 1 beschrieben. Die Menge der (C₄-Anteil) Fraktionen c+d beträgt ca. 91.000 jato.

### Beispiel 3

### Beispiel 3 wird durch Schema 3 weiter verdeutlicht.

375.000 jato eines Roh-C₄-Stroms aus einem Naphtha-Cracker werden komplett einer Selektivhydrierung zugeführt. Danach hat der Strom folgende Zusammensetzung: 33,3% Isobuten, 24% 1-Buten, 40% 2-Butene, 100 ppm 1,3-Butadien, Rest Butane. Dieser Feed wird einer Stufe zur selektiven Hydroformylierung von 1-Buten zugeführt. Der 1-Buten-Umsatz liegt bei 90%, insgesamt je 3,5% des 1-Butens werden dabei zu 2-Buten bzw. Butan umgesetzt. Es werden rund 115.000 jato Valeraldehyd produziert. Der verbleibende C₄-Strom (knapp 300.000 jato) besteht aus 41,7% Isobuten, 3% 1-Buten, 50,9% 2-Butene, 120 ppm 1,3-Butadien und Rest Butane. Dieser Feed wird destillativ aufgespalten: Das Kopfprodukt (136.000 jato) besteht aus 80,5% Isobuten, 5,8% 1-Buten, 9,5% 2-Butenen, 110 ppm 1,3-Butadien, Rest Butane. Das Sumpfprodukt (163.000 jato) besteht aus 9,3% Isobuten, 0,7% 1-Buten, 85,5% 2-Butenen, 10 ppm 1,3-Butadien, Rest Butane. Das Sumpfprodukt wird zunächst über ein 13X-Molsieb zur Entfernung von Oxygenatspuren geschickt und anschließend zusammen 70000 jato Ethylen der Metathesestufe zugeführt. Die Metathese läuft in der Flüssigphase an einem Festbettkatalysator, 10 wt% Re2O7 auf einem Al₂O₃-Träger. Die Temperatur wird zum Ausgleich des fortschreitenden Aktivitätsverlusts nachgeregelt und beträgt 40°C bei start of run und 120°C bei end of run. Wenn nach ca. 6 Tagen die Endtemperatur erreicht ist, wird der Katalysator oxidativ bei Temperaturen von ca. 550°C regeneriert. In dieser Zeit übernimmt ein zweiter, paralleler Reaktor (A/B-Fahrweise) die Produktion. Bei einem durchschnittlichen Gleichgewichtsumsatz von ca. 63% werden knapp 133.000 jato Propen produziert. Die Aufarbeitung des in der Metathesestufe gebildeten Stoffstroms erfolgt wie bei Schema 1 beschrieben. Die Menge an Fraktion (C4-Fraktion) C+d beträgt 75.000 jato.

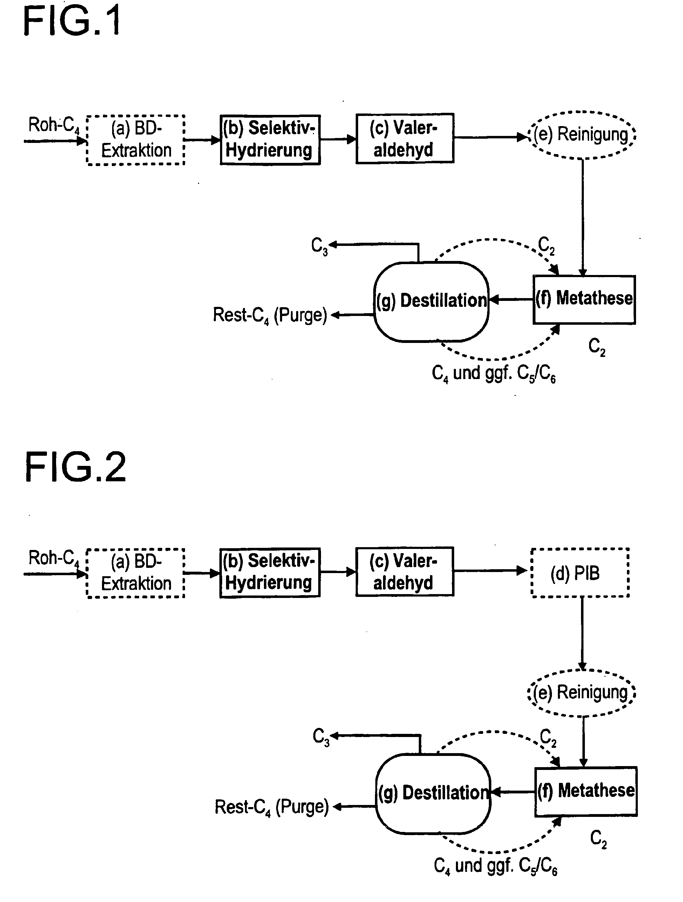

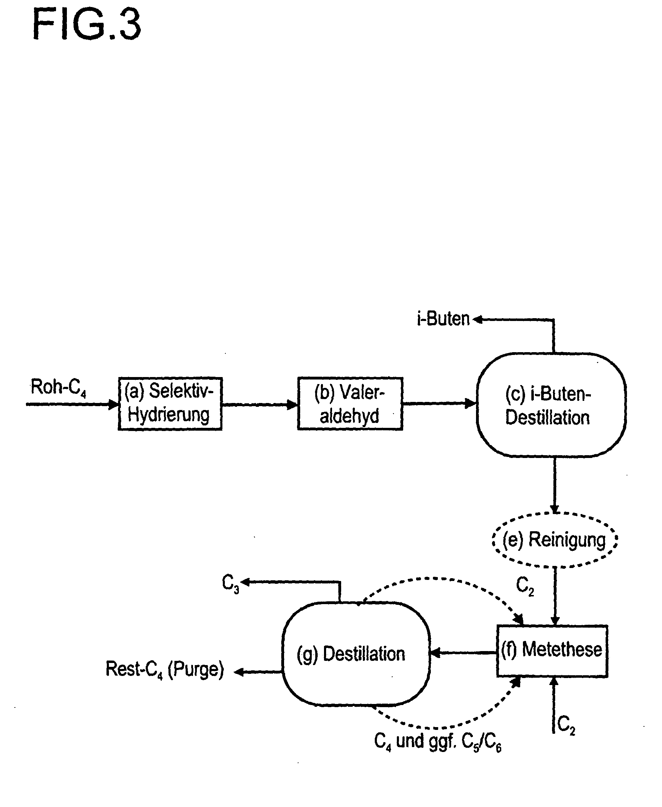

## Patentansprüche

1. Verfahren zur Herstellung von C₅-Aldehyden und Propen aus einem 1-Buten- und 2-Butene-haltigen C₄-Strom, der bis zu 1000 Gew.-ppm 1,3-Butadien enthält (C₄-Eingangsstrom), umfassend
a) eine Hydroformylierungsstufe, bei der man den C₄-Eingangsstrom in Gegenwart eines üblichen Hydroformylierungskatalysators mit Wasserstoff und Kohlenmonoxid in Kontakt bringt und die dabei gebildeten C₅-Aldehyde und den dabei gebildeten 2-Buten-reichen C₄-Strom anschließend voneinander trennt und
b) eine Metathesestufe, bei der man den in der Hydroformylierungsstufe gebildeten 2-Buten-reichen C₄-Strom in Gegenwart eines üblichen Metathesekatalysators mit Ethen in Kontakt bringt und von dem dabei gebildeten Propen-haltigen Kohlenwasserstoffstrom das Propen abtrennt.

2. Verfahren nach Anspruch 1, wobei man den C₄-Eingangsstrom bereitstellt, indem man
Ia) in Schritt Ia Naphtha oder sonstige Kohlenwasserstoffverbindungen einem Steamcracking- oder FCC-Prozess unterwirft und aus dem dabei gebildeten Stoffstrom eine C₄-Olefin-Mischung abzieht, die 1-Buten, 2-Buten, und mehr als 1000 Gew.-ppm Butadiene und ggf. Butine und ggf. Isobuten enthält und
IIa) aus der in Schritt Ia gebildeten C₄- Olefin-Mischung einen im wesentlichen aus, 1-Buten, 2-Butenen und ggf. Butanen und ggf. Isobuten bestehenden C₄-Kohlenwasserstoffstrom (Raffinat I) herstellt, indem man mittels Selektivhydrierung die Butadiene und Butine zu Butenen oder Butanen hydriert oder die Butadiene und Butine durch Extraktivdestillation soweit entfernt, dass der Gehalt an 1,3-Butadien maximal 1000 Gew.-ppm beträgt.

3. Verfahren nach Anspruch 1, wobei man den C₄-Eingangsstrom bereitstellt, indem man
Ib) in Schritt Ib aus einem Butane enthaltenden Kohlenwasserstoffstrom durch Dehydrierung und nachfolgende Reinigung eine C₄-Olefin-Mischung herstellt, die Isobuten, 1-Buten, 2-Butene, und mehr als 1000Gew.-ppm Butadiene und ggf. Butine und ggf. Butanen enthält 3.
IIb) aus der in Schritt IB gebildeten C₄-Olefin-Mischung einen im wesentlichen aus Isobuten, 1-Büten, 2-Butenen und ggf. Butanen bestehenden C4-Kohlenwasserstoffstrom (Raffinat I) herstellt, indem man mittels Selektivhydrierung die Butadiene und Butine zu Butenen oder Butanen hydriert oder die Butadiene und Butine durch Extraktivdestillation soweit entfernt, dass der Gehalt an 1,3-Butadien maximal 1000Gew.-ppm beträgt.

4. Verfahren nach Anspruch 2 oder 3, wobei man, sofern der Gehalt an 1,3-Butadien in der gemäß Schritt la oder Schritt Ib gewonnenen C₄-Olefin-Mischung 5 Gew.-% oder mehr beträgt, den Gehalt an 1,3-Butadien mittels Extraktivdestillation auf einen Gehalt zwischen1000 Gew.-ppm und 5 Gew.-% absenkt und den Gehalt an 1,3-Butadien anschließend mittels Selektivhydrierung weiter auf 1000Gew.-ppm oder weniger absenkt.

5. Verfahren nach einem der vorstehenden Ansprüche, umfassend eine ein- oder mehrstufige Reinigung des 2-Buten-reichen C₄-Stroms, geeignet zur Entfernung von Oxigenaten, oder Spuren von Diolefinen und Acetylenen, wobei man den 2-Buten-reichen C₄-Strom durch ein Adsorberbett mit einem Molsieb, Alumosilikat oder Aluminiumoxid führt.

6. Verfahren nach einem der vorstehenden Ansprüche, wobei der C₄-Eingangsstrom ein Verhältnis 1-Buten zu 2-Butene von 3 : 1 bis 1 : 3 aufweist.

7. Verfahren nach einem der vorstehenden Ansprüche, wobei das Verhältnis 1-Buten zu 2-Buten im 2-Buten-reichen C₄-Strom 1 : 3 bis 1 : 60 beträgt.

8. Verfahren nach einem der vorstehenden Ansprüche, wobei der C₄-Eingangsstrom 2 bis 50 Gew.-% Butane, 15 bis 80 Gew.-% 2-Butene und 20 bis 60 Gew.% 1-Buten, 20 bis 1000 Gew.-ppm Butadiene und 0 bis 50 Gew.-% Isobuten enthält.

9. Verfahren nach einem der vorstehenden Ansprüche, wobei der 2-Buten-reiche C₄-Strom 3 bis 70 Gew.-% Butane, 22 bis 90 Gew.-% 2-Butene, 20 bis 1000 Gew.-ppm 1,3-Butadien, 0 bis 10 Gew.-% 1-Buten und 0 bis 65 Gew.-% Isobuten enthält.

10. Verfahren nach einem der vorstehenden Ansprüche, wobei man, sofern der in der Metathesestufe gebildete Propen-haltigen Kohlenwasserstoffstrom C₅- und C₆-Olefine enthält, diese vom Propen abtrennt und zumindest in dem Umfang in die Metathesestufe zurückführt, dass das molare Verhältnis der Summe der nicht zurückgeführten C₅- und C₆-Olefine zu Propen mit maximal 0,2 : 1 beträgt.

11. Verfahren nach einem der vorstehenden Ansprüche, wobei man einen C₄-Eingangsstrom einsetzt, der Isobuten enthält und dessen relativen Isobutengehalt im C₄-Eingangsstrom oder im 2-Buten-reichen C₄-Strom um wenigstens 40% reduziert.

12. Verfahren nach Anspruch 11, wobei man die Reduktion des Isobutengehaltes des 2-Buten-reichen C₄-Strom vornimmt, indem man den 2-Buten-reichen C₄-Strom mit einem üblichen Katalysator für die Polymerisation von Isobuten in Kontakt bringt und das gebildete Polyisobutylen von dem verbleibenden 2-Buten-reichen C₄-Strom abtrennt.

13. Verfahren nach Anspruch 11, wobei man die Reduktion des Isobutengehaltes des C₄-Eingangsstrom oder des 2-Buten-reichen C₄-Strom vornimmt, indem man den C₄-Eingangsstrom bzw. den 2-Buten-reichen C₄-Strom mit einem Alkylalkohol und einem üblichen Katalysator für die Bildung von Alkyl-tert-butylether in Kontakt bringt und den gebildeten Alkyl-tert-butylether von dem verbleibenden C₄-Eingangsstrom bzw. den 2-Buten-reichen C₄-Strom abtrennt.

14. Verfahren nach Anspruch 11, wobei man die Reduktion des Isobutengehaltes des C₄-Eingangsstrom oder des 2-Buten-reichen C₄-Strom mittels Destillation vornimmt.

15. Verfahren nach einem der vorstehenden Ansprüche, wobei man die Abtrennung des Propen von dem in der Metathesestufe gebildeten Produktstrom durch Destillation vornimmt und das Propen dabei oder ggf. in einem darauf folgenden Destillationsstufe soweit reinigt, dass es der Spezifikation "polymer grade" entspricht.

## Claims

1. A process for preparing C₅ aldehydes and propene from a 1-butene- and 2-butenes-containing C₄ stream which comprises up to 1000 ppm by weight of 1,3-butadiene (C₄ starting stream), comprising
a) a hydroformylation stage in which the C₄ starting stream is contacted in the presence of a customary hydroformylation catalyst with hydrogen and carbon monoxide, and the thus formed C₅ aldehydes and the thus formed 2-butene-rich C₄ stream are subsequently separated from one another, and
b) a metathesis stage in which the 2-butene-rich C₄ stream formed in the hydroformylation stage is contacted with ethene in the presence of a customary metathesis catalyst and the propene is removed from the thus formed propene-containing hydrocarbon stream.

2. The process according to claim 1, wherein the C₄ starting stream is provided by
Ia) in step la, subjecting naphtha or other hydrocarbon compounds to a steamcracking or FCC process and drawing off from the thus formed stream a C₄ olefin mixture which comprises 1-butene, 2-butene and more than 1000 ppm by weight of butadienes, with or without butynes and isobutene, and
IIa) preparing from the C₄ olefin mixture formed in step la a C₄ hydrocarbon stream consisting substantially of 1-butene and 2-butenes, with or without butanes and isobutene (raffinate I), by hydrogenating the butadienes and butynes to butenes or butanes by means of selective hydrogenation, or removing the butadienes and butynes by extractive distillation to such an extent that the content of 1,3-butadiene is not more than 1000 ppm by weight.

3. The process according to claim 1, wherein the C₄ starting stream is prepared by
Ib) in step Ib, preparing from a hydrocarbon stream comprising butanes, by dehydrogenation and subsequent purification, a C₄ olefin mixture which comprises isobutene, 1-butene, 2-butenes and more than 1000 ppm by weight of butadienes, with or without butynes and butanes,
IIb) preparing from the C₄ olefin mixture formed in step Ib a C₄ hydrocarbon stream consisting substantially of isobutene, 1-butene and 2-butenes, with or without butanes (raffinate I), by hydrogenating the butadienes and butynes to butenes or butanes by means of selective hydrogenation, or removing the butadienes and butynes by extractive distillation to such an extent that the content of 1,3-butadiene is not more than 1000 ppm by weight,

4. The process according to claim 2 or 3, wherein, when the content of 1,3-butadiene in the C₄ olefin mixture obtained in step la or step 16 is 5% by weight or more, the content of 1,3-butadiene is lowered by means of extractive distillation to a content between 1000 ppm by weight and 5% by weight, and the content of 1,3-butadiene is subsequently lowered further by means of selective hydrogenation to 1000 ppm by weight or less.

5. The process according to any of the preceding claims, comprising a one- or multistage purification of the 2-butene-rich C₄ stream, suitable for removing oxygenates, or traces of diolefins and acetylenes, wherein the 2-butene-rich C₄ stream is conducted through an adsorber bed comprising a molecular sieve, aluminosilicate or alumina.

6. The process according to any of the preceding claims, wherein the C₄ starting stream has a ratio of 1-butene to 2-butenes of from 3 : 1 to 1 : 3.

7. The process according to any of the preceding claims, wherein the ratio of 1-butene to 2-butene in the 2-butene-rich C₄ stream is from 1 : 3 to 1 : 60.

8. The process according to any of the preceding claims, wherein the C₄ starting stream comprises from 2 to 50% by weight of butanes, from 15 to 80% by weight of 2-butenes and from 20 to 60% by weight of 1-butene, from 20 to 1000 ppm by weight of butadienes and from 0 to 50% by weight of isobutene.

9. The process according to any of the preceding claims, wherein the 2-butene-rich C₄ stream comprises from 3 to 70% by weight of butanes, from 22 to 90% by weight of 2-butenes, from 20 to 1000 ppm by weight of 1,3-butadiene, from 0 to 10% by weight of 1-butene and from 0 to 65% by weight of isobutene.

10. The process according to any of the preceding claims, wherein, when the propene-containing hydrocarbon stream formed in the metathesis stage comprises C₅ and C₆ olefins, they are removed from the propene and recycled into the metathesis stage at least to the extent that the molar ratio of the sum of the unrecycled C₅ and C₆ olefins to propene is not more than 0.2 : 1.

11. The process according to any of the preceding claims, wherein a C₄ starting stream is used which comprises isobutene and whose relative isobutene content in the C₄ starting stream or in the 2-butene-rich C₄ stream is reduced by at least 40%.

12. The process according to claim 11, wherein the reduction in the isobutene content of the 2-butene-rich C₄ stream is undertaken by contacting the 2-butene-rich C₄ stream with a customary catalyst for the polymerization of isobutene, and removing the polyisobutylene formed from the remaining 2-butene-rich C₄ stream.

13. The process according to claim 11, wherein the reduction in the isobutene content of the C₄ starting stream or of the 2-butene-rich C₄ stream is undertaken by contacting the C₄ starting stream or the 2-butene-rich C₄ stream with an alkyl alcohol and a customary catalyst for the formation of alkyl tert-butyl ether, and removing the alkyl tert-butyl ether formed from the remaining C₄ starting stream or the 2-butene-rich C₄ stream.

14. The process according to claim 11, wherein the reduction in the isobutene content of the C₄ starting stream or of the 2-butene-rich C₄ stream is undertaken by means of distillation.

15. The process according to any of the preceding claims, wherein the removal of the propene from the product stream formed in the metathesis stage is undertaken by distillation and the propene is purified thus or, if appropriate, in a subsequent distillation stage to such an extent that it corresponds to the polymer-grade specification.

## Revendications

1. Procédé de production d'aldéhydes en C₅ et de propène à partir d'un courant de C₄ contenant du 1-butène et des 2-butènes, qui contient jusqu'à 1000 ppm en poids de 1,3-butadiène (courant d'entrée de C₄), comportant :
a) une étape d'hydroformylation, dans laquelle le courant d'entrée de C₄ est mis en contact avec de l'hydrogène et du monoxyde de carbone en présence d'un catalyseur d'hydroformylation usuel, et les aldéhydes de C₅ formées à cette occasion et le courant de C₄ riche en 2-butène formé à cette occasion sont ensuite séparés l'un de l'autre et
b) une étape de métathèse, dans laquelle le courant de C₄ riche en 2-butène formé lors de l'étape d'hydroformylation est mis en contact avec de l'éthène en présence d'un catalyseur de métathèse usuel et le propène est isolé du courant d'hydrocarbures contenant du propène formé à cette occasion.

2. Procédé selon la revendication 1, dans lequel le courant d'entrée de C₄ est préparé en :
Ia) soumettant dans l'étape Ia du naphta ou d'autres composés hydrocarbures à un craquage à la vapeur ou à un procédé FCC (craquage catalytique en lit fluidisé), et en extrayant un mélange oléfinique de C₄ à partir du courant de matière formé à cette occasion, qui contient du 1-butène, du 2-butène, et plus de 1000 ppm en poids de butadiènes et éventuellement de butynes et éventuellement d'isobutène et
IIa) en produisant un courant d'hydrocarbures de C₄ (raffinat I) constitué essentiellement de 1-butène, de 2-butènes, et éventuellement de butanes et éventuellement d'isobutène à partir du mélange oléfinique de C₄ formé dans l'étape Ia, en hydrogénant les butadiènes et les butynes pour donner des butènes ou des butanes au moyen de l'hydrogénation sélective, ou en éliminant les butadiènes et les butynes grâce à la distillation extractive, jusqu'à ce que la teneur en 1,3-butadiène soit au maximum de 1000 ppm en poids.

3. Procédé selon la revendication 1, dans lequel le courant d'entrée de C₄ est préparé en :
Ib) produisant dans l'étape Ib un mélange oléfinique de C₄ qui contient de l'isobutène, du 1-butène, des 2-butènes, et plus de 1000 ppm en poids de butadiènes et éventuellement des butynes et éventuellement des butanes, à partir d'un courant d'hydrocarbures contenant des butanes grâce à une déshydrogénation et un raffinage consécutif, et
IIb) en produisant un courant d'hydrocarbures de C₄ (raffinat I) constitué essentiellement d'isobutène, de 1-butène, de 2-butènes, et éventuellement de butanes à partir du mélange oléfinique de C₄ formé dans l'étape Ib, en hydrogénant les butadiènes et les butynes pour donner des butènes ou des butanes au moyen de l'hydrogénation sélective, ou en éliminant les butadiènes et les butynes grâce à la distillation extractive, jusqu'à ce que la teneur en 1,3-butadiène soit au maximum de 1000 ppm en poids.

4. Procédé selon la revendication 2 ou 3, dans lequel la teneur en 1,3-butadiène est abaissée au moyen de la distillation extractive jusqu'à une teneur comprise entre 1000 ppm en poids et 5 % en poids, dans la mesure où la teneur en 1,3-butadiène dans le mélange oléfinique de C₄ obtenu dans l'étape Ia ou dans l'étape Ib est de 5 % en poids ou plus, et la teneur en 1,3-butadiène est encore abaissée ensuite au moyen de l'hydrogénation sélective jusqu'à 1000 ppm en poids ou moins.

5. Procédé selon l'une quelconque des revendications précédentes, comportant un raffinage en une ou plusieurs étapes du courant de C₄ riche en 2-butène, approprié pour l'élimination d'oxygénats, ou de traces de dioléfines et d'acétylènes, le courant de C₄ riche en 2-butène étant guidé à travers un lit d'adsorption muni d'un tamis moléculaire, de l'aluminosilicate ou de l'oxyde d'aluminium.

6. Procédé selon l'une quelconque des revendications précédentes, dans lequel le courant d'entrée de C₄ présente un rapport 1-butène sur 2-butènes de 3:1 à 1:3.

7. Procédé selon l'une quelconque des revendications précédentes, dans lequel le rapport 1-butène sur 2-butène dans le courant de C₄ riche en 2-butène est de 1:3 à 1:60.

8. Procédé selon l'une quelconque des revendications précédentes, dans lequel le courant d'entrée de C₄ contient 2 à 50% en poids de butanes, 15 à 80 % en poids de 2-butènes et 20 à 60 % en poids de 1-butène, 20 à 1000 ppm en poids de butadiènes et 0 à 50 % en poids d'isobutène.

9. Procédé selon l'une quelconque des revendications précédentes, dans lequel le courant de C₄ riche en 2-butène contient 3 à 70 % en poids de butanes, 22 à 90 % en poids de 2-butènes, 20 à 1000 ppm en poids de 1,3-butadiène, 0 à 10 % en poids de 1-butène et 0 à 65 % en poids d'isobutène.

10. Procédé selon l'une quelconque des revendications précédentes, dans lequel le courant d'hydrocarbures contenant du propène formé dans l'étape de métathèse est isolé du propène et est remis en circulation dans l'étape de métathèse dans la mesure où le rapport molaire de la somme des oléfines C₅ et des oléfines C₆ non remises en circulation sur le propène est au maximum de 0,2:1, si tant est que ce courant contient des oléfines C₅ et des oléfines C₆.

11. Procédé selon l'une quelconque des revendications précédentes, dans lequel un courant d'entrée de C₄ est utilisé, qui contient de l'isobutène et dont la teneur relative en isobutène dans le courant d'entrée de C₄ ou dans le courant de C⁴ riche en 2-butène est réduite d'au moins 40 %.

12. Procédé selon la revendication 11, dans lequel la réduction de la teneur en isobutène du courant de C₄ riche en 2-butène est entreprise en mettant le courant de C₄ riche en 2-butène en contact avec un catalyseur usuel pour la polymérisation de l'isobutène, et en isolant le polyisobutylène formé du courant de C₄ restant riche en 2-butène.

13. Procédé selon la revendication 11, dans lequel la réduction de la teneur en isobutène du courant d'entrée de C₄ ou du courant de C₄ riche en 2-butène est entreprise en mettant en contact le courant d'entrée de C₄ ou le courant de C₄ riche en 2-butène avec un alcool alkylique et un catalyseur usuel pour former de l'éther alkylique de tert-butyle, et en isolant l'éther alkylique de tert-butyle formé du courant d'entrée de C₄ restant ou du courant de C₄ riche en 2-butène.

14. Procédé selon la revendication 11, dans lequel la réduction de la teneur en isobutène du courant d'entrée de C₄ ou du courant de C₄ riche en 2-butène est entreprise au moyen d'une distillation.

15. Procédé selon l'une quelconque des revendications précédentes, dans lequel l'isolement du propène à partir du courant de produit formé dans l'étape de métathèse est entrepris grâce à une distillation et le propène est raffiné à cette occasion ou éventuellement dans une étape de distillation consécutive jusqu'à ce qu'il corresponde à la spécification "qualité polymère".
